# EUROPEAN PATENT SPECIFICATION

(11) **EP 1 747 017 B1**
(45) Date of publication and mention of the grant of the patent: **26.03.2008**
(21) Application number: 05746977.7
(22) Date of filing: 10.05.2005
(51) Int. Cl.: A61K 47/18, A61K 47/22, A61K 47/10

(54) **USE OF TARGETED OXIDATIVE THERAPEUTIC FORMULATION IN TREATMENT OF AGE-RELATED MACULAR DEGENERATION**
VERWENDUNGEN EINER TARGETIERTEN THERAPEUTISCHEN FORMULIERUNG FUR DIE BEHANDLUNG VON ALTERSBEDINGTER MAKULADEGENERATION
UTILISATION D'UNE FORMULATION THERAPEUTIQUE OXYDATIVE CIBLEE DANS LE TRAITEMENT DE LA DEGENERESCENCE MACULAIRE LIEE A L'AGE

(30) Priority: 10.05.2004 US 569792 P
(43) Date of publication of application: 31.01.2007
(73) Proprietor: Hofmann, Robert F., Austin, TX 78746-2432 (US)
(72) Inventor: Hofmann, Robert F., Austin, TX 78746-2432 (US)
(74) Representative: Fisher, Adrian John
(86) International application number: PCT/US2005/016161
(87) International publication number: WO 2005/110484

(56) References cited:
- WO-A-02/078622
- WO-A-02/078623
- US-A- 5 190 979
- US-A- 5 270 344

## Description

### BACKGROUND

This application claims priority to U.S. Provisional Patent Application Serial Number 60/569,792, entitled "Use of Targeted Oxidative Therapeutic Formulation in Treatment of Age-Related Macular Degeneration" filed on May 10, 2004.

The present invention relates to a composition containing peroxidic species or oxidation products, its method of preparation, and its use. More specifically, the invention relates to a pharmaceutical composition or formulation which contains: peroxidic species or reaction products resulting from oxidation of an olefinic compound, in a liquid form or in a solution, by an oxygen-containing oxidizing agent; a penetrating solvent; a dye containing a chelated metal; and an aromatic redox compound. The invention also relates to the preparation of the pharmaceutical formulation and its use in treating age-related macular degeneration.

Age-related macular degeneration ("ARMD") is the leading cause of permanent vision loss for individuals over age 65, currently affecting 13 million Americans. ARMD affects light-sensitive photoreceptor cells and pigmented epithelial cells in the macula, the center of the retina of the eye. While it may not cause total blindness, the disease destroys central vision, making reading, watching TV and driving impossible. It has no documented cure, has never demonstrated spontaneous remission, and effective treatments are very limited.

The retina is a complicated network of nerve cells that changes light into nerve impulses that travel to the brain where they are interpreted as visual images. The central part of the retina, called the macula, is responsible for vision that is needed for reading and other detailed work. Damage to the macula results in poor vision. The most common disease process that affects the macula is ARMD. In patients with ARMD, retinal photoreceptor and pigment epithelial cells in the macula die over the course of several years. The cell death and gradual visual loss usually do not begin until age 60 or older, hence the name age-related macular degeneration.

There are two types of ARMD: dry macular degeneration and wet macular degeneration. Dry macular degeneration, although more common, typically results in a less severe, more gradual loss of vision. Patients who are affected by dry ARMD have gradual loss of central vision due to the death of photoreceptor cells and their close associates, retinal pigmented epithelial ("RPE") cells, with deposition of a complex waxy amyloid mixture, termed "drusen". Photoreceptors, the cells in the retina that actually "see" light, are essential for vision. Macrophagic RPE cells are necessary for photoreceptor survival, function and renewal.

Patients with wet macular degeneration develop new blood vessels under the retina. As the photoreceptor and RPE cells slowly degenerate, there is a tendency for blood vessels to grow from their normal location in the choroid into an abnormal location beneath the retina. This abnormal new blood vessel growth is called choroidal neovascularization ("CNV"). The abnormal blood vessels leak and bleed, causing hemorrhage, swelling, scar tissue, and severe loss of central vision. Only 10% of patients with ARMD have the wet type, but it is responsible for 90% of all blindness resulting from ARMD.

Depending on the location, laser treatment can sometimes be given to destroy the abnormal blood vessels formed in wet ARMD. Only 15% of the cases of wet ARMD are eligible to have laser treatment because the blood vessels can not be located too close to the center part of the macula. The laser is a beam of light that is absorbed by the pigment of blood, drugs and RPE cells, which converts to heat energy that cauterizes the abnormal blood vessels. Frequently the neovascularization returns, since the stimulus has not been removed, resulting in severe loss of vision. In fact, most of the patients with ARMD, who have very poor vision, have lost it due to sequelae of neovascularization. Current medical opinion states that there is no treatment available that permanently prevents the cell death or abnormal blood vessel growth that occurs in ARMD.

The RPE cells in the eye act as macrophages, which phagocytize and recycle components of the membranous outer segments of photoreceptors. If the mitochondria within the RPE cells are damaged, the photoreceptor recycling is inhibited, with resultant accumulation of the amorphous cellular debris called drusen.

Several studies have established a link between smoking and ARMD. Past and current smokers are at a four-times higher risk than non-smokers for developing ARMD. The effects of smoking upon ARMD development are so strong that the risk for former smokers can remain elevated beyond twenty years after the cessation of smoking. The consistency of association across populations with differing lifestyles and exposures all support a causal relationship between smoking and ARMD.

This causal relationship may be due to the effects of cadmium in the human body. On average, smokers have 4-5 times higher blood cadmium concentrations and 2-3 times higher kidney cadmium concentrations than non-smokers. Long-term exposure to the toxic metal substance has been linked to cellular immune deficiency, atherosclerosis, cancer, macular degeneration, and emphysema. Cadmium's toxicity is related to the damaged interaction of intracellular microfilaments ("MF"), whose assembly, linkages and disassembly are regulated by a number of calcium-dependent factors in mitochondria. Cadmium chloride causes MF denaturation in the form of irregular, disordered MF clots or plaques (DalleDonne *et al.,* 1997). Disruption of the MF causes a peroxidation deficit and the paralysis of phagocytosis. As a result, cellular debris accumulates as amyloid, drusen or plaques, eventually damaging tissue parenchyma cells, such as the photoreceptors. Persistent dysplasia of phagocytic cells eventually triggers proliferation, neoplasia and neovascularization (Levy *et al.,* 1986).

What is needed, therefore, is a means for restoring peroxidative activity and macrophage responsiveness in the RPE of individuals affected with ARMD.

Ozone is a triatomic gas molecule and an allotropic form of oxygen. It may be obtained by means of an electrical discharge or intense ultraviolet light through pure oxygen. The popular misconception that ozone is a serious pollutant, the "free radical" theory of disease, and the antioxidant supplement market have comprehensibly prejudiced medical orthodoxy against its use as a treatment. Ozone therapy, however, is a misnomer. Ozone is an extremely reactive and unstable gas with mechanisms of action directly related to the byproducts that it generates through selective interaction with organic compounds present in the plasma and in the cellular membranes. The selective reaction of ozone with unsaturated olefins occurs at the carbon-carbon double bond, generating ozonides. Ozone is toxic by itself, and its reaction products, ozonides, are unstable and are not therapeutic by themselves.

Hydrogen peroxide (H₂0₂), discovered in 1818, is present in nature in trace amounts. Hydrogen peroxide is unstable and decomposes violently (or foams) when in direct contact with organic membranes and particulate matter. Light, agitation, heating, and iron all accelerate the rate of hydrogen peroxide decomposition in solution. Hydrogen peroxide by direct contact *ex vivo* kills microbes that have low levels of peroxide-destroying enzymes, such as the catalases. However, there is no bactericidal effect when hydrogen peroxide is infused into the blood of rabbits infected with peroxide-sensitive *E. coli.* Moreover, increasing the concentration of peroxide *ex-vivo* in rabbit or human blood containing *E. coli* produces no evidence of direct bactericidal activity. The lack of effect of high concentrations of hydrogen peroxide is directly related to the presence of the peroxide-destroying enzyme catalase in the host animal's blood. To have any effect, high concentrations of hydrogen peroxide have to be in contact with the bacteria for significant periods of time. Large amounts of hydrogen peroxide-destroying enzymes, such as catalase, normally present in the blood make it impossible for peroxide to exist in blood for more than a few seconds. Thus, hydrogen peroxide introduced into the blood stream by injection or infusion does not directly act as an extracellular germicide in blood or extracellular fluids.

However, hydrogen peroxide does participate in the bactericidal processes of activated macrophage cells. Activated macrophage cells are drawn to the site of infection, attach to the infectious organism, and ingest it. The killing of the organisms takes place inside the macrophage cell by hydrogen peroxide. Hydrogen peroxide oxidizes cellular chloride to the chlorine dioxide free radical, which destabilizes microbial membranes and, if persistent, induces apoptosis or cellular suicide. The critical therapeutic criteria for intracellular peroxidation are the selective delivery, absorption and activation of peroxidic carrier molecules into only diseased macrophages, which are believed to be incapable of upgraded catalase and glutathione reductase activity. Infused hydrogen peroxide is a generalized poison whereas targeted intracellular peroxidation is a selective therapeutic tool.

Macrophage cells play critical roles in immunity, bone calcification, vision, neural insulation (myelinization), detoxification, pump strength, and clearance of toxins from the body, depending upon their site of localization. The energy requirements of macrophages are met by intracellular structures called mitochondria. Mitochondria are often structurally associated with the microfilament internal cytoarchitecture. The folded internal layer of the mitochondria creates the high-energy molecule ATP, while the outer layer contains cytochromes and electron recycling molecules that generate peroxides. The outer layers of mitochondria are susceptible to toxic blockade or damage by endotoxins, mycotoxins, virally encoded toxins, drugs, heavy metals, and pesticides. When the peroxidation function of mitochondria is blocked, the filament architecture of the cell tends to cross-link, generating incorrect signals, incompetence, inappropriate replication, or premature cell death.

U.S. Patent No. 4,451,480 to De Villez teaches a composition and method for treating acne. The method includes topically treating the affected area with an ozonized material derived from ozonizing various fixed oil and unsaturated esters, alcohols, ethers and fatty acids.

U.S. Patent No. 4,591,602 to De Villez shows an ozonide ofJojoba used to control microbial infections.

U.S. Patent No. 4,983,637 to Herman discloses a method to parenterally treat local and systemic viral infections by administering ozonides of terpenes in a pharmaceutically acceptable carrier.

U.S. Patent No. 5,086,076 to Herman shows an antiviral composition containing a carrier and an ozonide of a terpene. The composition is suitable for systemic administration or local application.

U.S. Patent No. 5,126,376 to Herman describes a method to topically treat a viral infection in a mammal using an ozonide of a terpene in a carrier.

U.S. Patent No. 5,190,977 to Herman teaches an antiviral composition containing a non-aqueous carrier and an ozonide of a terpene suitable for systemic injection.

U.S. Patent No. 5,190,979 to Herman describes a method to parenterally treat a medical condition in a mammal using an ozonide of a terpene in a carrier.

U.S. Patent No. 5,260,342 to Herman teaches a method to parenterally treat viral infections in a mammal using an ozonide of a terpene in a carrier.

U.S. Patent No. 5,270,344 to Herman shows a method to treat a systemic disorder in a mammal by applying to the intestine of the mammal a trioxolane or a diperoxide derivative of an unsaturated hydrocarbon which derivative is prepared by ozonizing the unsaturated hydrocarbon dissolved in a non-polar solvent.

U.S. Patent No. 5,364,879 to Herman describes a composition for the treatment of a medical condition in a mammal, the composition contains a diperoxide or trioxolane derivative of a non-terpene unsaturated hydrocarbon which derivative is prepared by ozonizing below 35° C the unsaturated hydrocarbon in a carrier.

WO02/07 describes oxidative therapeutic formulations containing peroxidic species or a reaction product resulting from oxidation of an alkene by a oxygen-containing oxidizing agent, a penetrating solvent, a dye containing a chelated metal, and a aromatic redox compound.

Despite the reports on the use of terpene ozonides for different medical indications, terpene ozonides display multiple deficiencies. For example, ozonides of monoterpene, such as myrcene and limonene, flamed out in the laboratory. Consequently, they are extremely dangerous to formulate or store.

Furthermore, ozonides of geraniol, a linear monoterpene alcohol, in water or in dimethylsulfoxide ("DMSO") did not show any clinical efficacy in three cases of viral Varicella Zoster (shingles) and two cases of Herpes Simplex dermatitis.

Thus, there is a need for a safe and effective pharmaceutical formulation or composition utilizing reaction products from the oxidation of an alkene compound. What is also needed is a method for stimulating mitochondrial defenses against free radical formation in RPE cells, thereby reducing the drusen and improving the visual acuity of individuals affected with ARMD.

### SUMMARY

This invention is directed to pharmaceutical formulations comprising peroxidic species or reaction products resulting from oxidation of an unsaturated organic compound, in a liquid form or in a solution, by an oxygen-containing oxidizing agent; a penetrating solvent; a chelated dye; and an aromatic redox compound. In one embodiment of the pharmaceutical formulation, the essential components include the peroxidic products formed by ozonolysis of an unsaturated alcohol, a stabilizing solvent, metalloporphyrin, and quinone. This invention is also directed to use of the pharmaceutical formulation to treat age-related macular degeneration ("ARMD").

The peroxidic species or reaction products are preferably formed through the reaction of an alkene and ozone. It is generally accepted that the reaction between an alkene and ozone proceeds by the Criegee mechanism. According to this mechanism, shown in Scheme 1 below, the initial step of the reaction is a 1,3-dipolar cycloaddition of ozone to the alkene to give a primary ozonide (a 1,2,3-trioxalane). The primary ozonide is unstable, and undergoes a 1,3-cycloreversion to a carbonyl compound and a carbonyl oxide. In the absence of other reagents or a nucleophilic solvent, this new 1,3-dipole enters into a second 1,3-dipolar cycloaddition to give the "normal" ozonide, a 1,2,4-trioxalane.

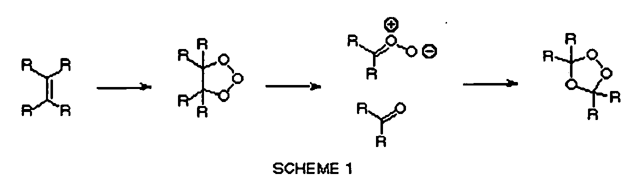

In a side reaction, the carbonyl oxide can enter into a dimerization to give a peroxidic dimer, the 1,2,4,5-tetraoxane, shown in Scheme 2 below.

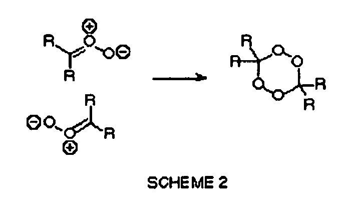

The carbonyl oxide is a strongly electrophilic species, and in the presence of nucleophilic species (e.g. alcohols or water), it undergoes facile nucleophilic addition to give a 1-alkoxyhydroperoxide, shown in Scheme 3 below. Under certain conditions, the 1-alkoxyhydroperoxide can undergo further reaction to give carboxylic acid derivatives.

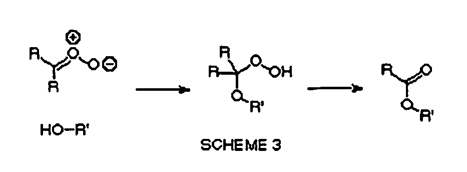

Again, not wanting to be bound by theory, it is believed that during the ozonolysis of the alcohol-containing alkene in the present invention, it is reasonable to expect that three major types of peroxidic products will be present: the normal ozonide, the carbonyl tetraoxane dimer, and the 1-alkoxyhydroperoxide. In the presence of water, some of these peroxidic products may also lead to the presence of organic peracids in the crude product mixture.

The present invention also involves the use of a penetrating solvent such as dimethylsulfoxide ("DMSO") to "stabilize" the initial products of the ozonolysis. Similarly, not wanting to be bound by any theory, it is believed that the stabilization is most likely a simple solvation phenomenon. However, DMSO is known to be a nucleophile in its own right. Its participation is also possible as a nucleophilic partner in stabilizing reactive species (for example, as dimethylsulfoxonium salts). The stabilized peroxidic molecule and the penetrating solvent of the current pharmaceutical formulation are made from components generally regarded as safe ("GRAS").

Another component of the pharmaceutical formulation is a chelated dye, such as a porphyrin. The propensity of metalloporphyrins to sensitize oxygen under photochemical excitation is well documented, as is the propensity of ferroporphyrins and copper porphyrins to bind oxygen-containing systems.

A further component of the pharmaceutical formulation is an aromatic redox compound, such as a quinone.

Although not wanting to be bound by any theory, it is postulated that the preferred pharmaceutical formulation is a combination of biochemical agents that induce recycling autocatalytic oxidation in infected or dysplastic macrophages. The pharmaceutical formulation stimulates targeted apoptosis (cell suicide) through unopposed peroxidation. Thus, the pharmaceutical formulation creates therapeutic effects in a number of seemingly disparate mitochondria-based macrophagic diseases. In particular, the pharmaceutical formulation has been shown to be effective in reducing whole body insulin resistance, lowering blood glucose response, and improving muscle glucose uptake, which indicates its effectiveness at treating diabetes and obesity.

### DETAILED DESCRIPTION OF PREFERRED EMBODIMENTS

The current invention pertains to pharmaceutical formulations comprising peroxidic species or reaction products resulting from oxidation of an unsaturated organic compound, in a liquid form or in a solution, by an oxygen-containing oxidizing agent; a penetrating solvent; a chelated dye; and an aromatic redox compound. The pharmaceutical formulations may be used to treat individuals affected with age-related macular degeneration ("ARMD"). In one embodiment of the present invention, the essential components of the pharmaceutical formulation include the peroxidic products formed by ozonolysis of an unsaturated alcohol, a stabilizing solvent, metalloporphyrin, and quinone.

The unsaturated organic compound, which may also be an unsaturated olefinic hydrocarbon, of the pharmaceutical formulation can be an alkene without a hydroxyl group, or a hydroxyl-containing alkene. Preferably, the alkene has less than about 35 carbons. The alkene without a hydroxyl group may be an open-chain unsaturated hydrocarbon, a monocyclic unsaturated hydrocarbon, or a bicyclic unsaturated hydrocarbon. The hydroxyl-containing alkene can be an open-chain unsaturated alcohol, a monocyclic unsaturated alcohol, or a bicyclic unsaturated alcohol. The alkene may also be contained in a fixed oil, an ester, a fatty acid, or an ether.

Usable unsaturated olefinic hydrocarbons may be unsubstituted, substituted, cyclic or complexed alkenes, hydrazines, isoprenoids, steroids, quinolines, carotenoids, tocopherols, prenylated proteins, or unsaturated fats. The preferred unsaturated hydrocarbons for this invention are alkenes and isoprenoids.

Isoprenoids are found primarily in plants as constituents of essential oils. While many isoprenoids are hydrocarbons, oxygen-containing isoprenoids also occur such as alcohols, aldehydes, and ketones. In a formal sense, the building block of isoprenoid hydrocarbons may be envisaged as the hydrocarbon isoprene, CH₂=C(CH₃)-CH=CH₂, although it is known that isoprene itself is an end-product of isoprenoid biosynthesis and not an intermediate. Isoprenoid hydrocarbons are categorized by the number of isoprene (C₅H₈) units they contain. Thus, monoterpenes have 2, sesquiterpenes have 3, diterpenes have 4, sesterterpenes have 5, triterpenes have 6, and tetraterpenes have 8 isoprene units, respectively. Tetraterpenes are much more commonly known as carotenoids.

Limonene and pinene are examples of a monoterpene. Farnesol and nerolidol are examples of a sesquiterpene alcohol. Vitamin A₁ and phytol are examples of a diterpene alcohol while squalene is an example of a triterpene. Provitamin A₁, known as carotene, is an example of a tetraterpene. Geraniol, a monoterpene alcohol, is liquid in both its oxygen bound and normal states and is safe to living cells.

Preferred unsaturated hydrocarbons for the pharmaceutical formulation include alkene isoprenoids, such as myricene, citrillene, citral, pinene, or limonene. Preferred unsaturated hydrocarbons also include linear isoprenoid alcohols with two to four repeating isoprene groups in a linear chain, such as terpineol, citronellol, nerol, phytol, menthol, geraniol, geranylgeraniol, linalool, or farnesol.

The unsaturated organic compound may be linear, branched, cyclic, spiral, or complexed with other molecules in its configuration. The unsaturated organic compound may naturally exist in a gaseous liquid or solid state prior to binding with the oxidizing agent.

An open-chain unsaturated hydrocarbon can be: CₙH₂ₙ, one double bond, n=2-20; CₙH₂ₙ₋₂, two double bonds, n=4-20; CₙH₂ₙ₋₄, three double bonds, n=6-20; CₙH₂ₙ₋₆, four double bonds, n=8-20; C₂₅H₄₀, sesterterpene hydrocarbon; or C₃₀H₄₈, triterpene hydrocarbon.

A monocyclic unsaturated hydrocarbon can be: CₙH₂ₙ₋₂, one double bond and one ring, n=3-20; CₙH₂ₙ₋₄, two double bonds and one ring, n=5-20; CₙH₂ₙ₋₆, three double bonds and one ring, n=7-20; C₂₅H₄₀, sesterterpene hydrocarbon; or C₃₀H₄₈, triterpene hydrocarbon.

A bicyclic unsaturated hydrocarbon can be: CₙH₂ₙ₋₄, one double bond and two rings, n=4-20; CₙH₂ₙ₋₆, two double bonds and two rings, n=6-20; C₂₅H₄₀, sesterterpene hydrocarbon; or C₃₀H_{48,} triterpene hydrocarbons.

An open-chain unsaturated alcohol can be: CₙH₂ₙOₘ, one double bond, n=3-20, m=1-4; CₙH₂ₙ₋₂Oₘ, two double bonds, n=5-20, m=1-4; CₙH2ₙ-4Oₘ, three double bonds, n=7-20, m=1-4; CₙH₂ₙ₋₆Oₘ, four double bonds, n=9-20, m=1-4; C₂₅H₄₀Oₘ, m=1-4, sesterterpene alcohols; or C₃₀H₄₈Oₘ, m=1-4, triterpene alcohols.

A monocyclic unsaturated alcohol can be: CₙH₂ₙ₋₂Oₘ, one double bond and one ring, n=3-20, m=1-4; CₙH₂ₙ₋₄Oₘ, two double bonds and one ring, n=5-20, m=1-4; CₙH₂ₙ₋₆Oₘ, three double bonds + one ring, n=7-20, m=1-4; C₂₅H₄₀Oₘ, m=1-4, sesterterpene alcohols; or C₃₀H₄₈Oₘ, m=1-4, triterpene alcohols.

A bicyclic unsaturate alcohol can be: CₙH₂ₙ₋₄Oₘ, one double bond and two rings, n=5-20, m=1-4; CₙH₂ₙ₋₆Oₘ, two double bonds and two rings, n=7-20, m=1-4; C₂₅H₄₀Oₘ m=1-4, sesterterpene alcohols; or C₃₀H₄₈Oₘ, m=1-4, triterpene alcohols.

Based on the total weight of the pharmaceutical formulation, the alkene can vary from about 0.001% to about 30%, preferably from about 0.1% to about 5.0%, and more preferably from about 0.5% to about 3.0%.

The oxygen-containing oxidizing agent of the pharmaceutical formulation, which oxidizes the unsaturated hydrocarbon, may be singlet oxygen, oxygen in its triplet state, superoxide anion, ozone, periodate, hydroxyl radical, hydrogen peroxide, alkyl peroxide, carbamyl peroxide, benzoyl peroxide, or oxygen bound to a transition element, such as molybdenum (e.g. MoO₅).

The preferred method to bind "activated oxygen" to intact an isoprenoid alcohol, such as geraniol, is by ozonation at temperatures between 0-20°C in the dark in the absence of water or polar solvent. The geraniol "ozonides" are then dissolved and stabilized in 100% DMSO in the dark to prevent premature breakdown of the products. Although not wanting to be bound by any theory, it is believed that the catalytic breakdown of the tetraoxane peroxidic dimer byproduct of geraniol ozonation, which is not an ozonide, occurs inside of cells in the presence of superoxide anion. The final reactive therapeutic agents released are hydrogen peroxide and acetic acid.

The pharmaceutical formulation also utilizes a penetrating solvent. The penetrating solvent, which stablizes the oxygen-bound unsaturated hydrocarbon, may be an emollient, a liquid, a liposome, a micelle membrane, or a vapor. Usable penetrating solvents include aqueous solution, fats, sterols, lecithins, phosphatides, ethanol, propylene glycol, methylsulfonylmethane, polyvinylpyrrolidone, pH-buffered saline, and dimethylsulfoxide ("DMSO"). The preferred penetrating solvents include DMSO, polyvinylpyrrolidone, and pH-buffered saline. The most preferred penetrating solvent is DMSO.

Based on the total weight of the pharmaceutical formulation, the penetrating solvent can vary from about 50% to about 99%, preferably from about 90% to about 98%, and more preferably from about 95% to about 98%.

The "stabilized" peroxidic molecule and its penetrating solvent have been made from components currently used in production regulated by the Food and Drug Administration ("FDA"). These ingredients are the subject of Drug Master Files, Drug Monographs, are found in the USP/NF, or are Generally Recognized As Safe ("GRAS").

Another component of the pharmaceutical formulation is a chelated dye. The dye preferably contains a chelated divalent or trivalent metal, such as iron, copper, manganese tin, magnesium, or strontium. The preferred chelated metal is iron. The propensity of chelated dyes such as metalloporphyrins to sensitize oxygen under photochemical excitation is well documented, as is the propensity of ferroporphyrins and copper porphyrins to bind oxygen-containing systems. Usable dyes include natural or synthetic dyes. Examples of these dyes include porphyrins, rose bengal, chlorophyllins, hemins, porphins, corrins, texaphrins, methylene blue, hematoxylin, eosin, erythrosin, flavinoids, lactoflavin, anthracene dyes, hypericin, methylcholanthrene, neutral red, phthalocyanine, fluorescein, eumelanin, and pheomelanin. Preferred dyes can be any natural or synthetic porphyrin, hematoporphyrin, chlorophyllin, rose bengal, their respective congeners, or a mixture thereof. The most preferred dyes are naturally occurring porphyrins, such as hematoporphyrin, and rose bengal. The dye may be responsive to photon, laser, ionizing radiation, phonon, electrical cardiac pulse, electroporation, magnetic pulse, or continuous flow excitation.

Based on the total weight of the pharmaceutical formulation or composition, the dye can vary from about 0.1% to about 30%, preferably from about 0.5% to about 5%, and more preferably from about 0.8% to about 1.5%.

A further component of the pharmaceutical formulation is an aromatic redox compound, such as a quinone. The aromatic redox compound may be any substituted or unsubstituted benzoquinone, naphthoquinone, or anthroquinone. Preferred aromatic redox compounds include benzoquinone, methyl-benzoquinone, naphthoquinone, and methyl-naphthoquinone. The most preferred aromatic redox compound is methyl-naphthoquinone.

Based on the total weight of the pharmaceutical formulation, the aromatic redox compound can vary from about 0.01% to about 20.0%, preferably from about 0.1% to about 10%, and more preferably from about 0.1% to about 0.5%.

The pharmaceutical formulation is also preferably activated by an energy source or an electron donor. Useful electron donors include an electrical current, ascorbate or ascorbic acid, NADH, NADPH and germanium sesquioxide. Preferred electron donors include ascorbate and germanium sesquioxide. The most preferred electron donor is ascorbic acid in any salt form.

Based on the total weight of the pharmaceutical formulation, the electron donor can vary from about 0.01% to about 20%, preferably from about 1% to about 10%, and more preferably from about 1% to about 5%.

In order to obtain a biological effect *in vivo,* the pharmaceutical formulation is preferably infused as an ozonolysis-generated peroxidic product of an unsaturated hydrocarbon, rather than an ozonide, in conjunction with a superoxide generating chelated dye and an aromatic quinone. The unsaturated hydrocarbon product, or peroxidic dimer molecule, should be stabilized in a non-aqueous stabilizing solvent and should be capable of penetrating lipid membranes.

Researchers of energetically activated dye therapy have long known that the superoxide generating dye and the aromatic redox compound preferentially absorb into infected and dysplastic cells, which are typically also catalase deficient. Without wanting to be bound by theory, the catalase-induced destruction of peroxide should be overwhelmed in the target cells either naturally or by the pharmaceutical formulation. The peroxidic dimer should also be activated by the superoxide generating dye, initiating electron donation to the dimer and causing the release of hydrogen peroxide and acetic acid intracellularly. The electronic activation of the dye does not always require light, but rather may occur through small electrical pulses provided by, for example, a heart pulse. The peroxidation reaction within the infected macrophage then tends to destroy the prenylated protein linkage of microtubules within the cell, to destroy the infecting toxin, or to induce apoptosis of the macrophage host cell.

The pharmaceutical formulation is a combination of stable ingredients. These ingredients may preferably be stored as dry solid ingredients and liquid ingredients in separate containers, which are then mixed at the site of use. The dry solid ingredients preferably comprise the chelated dye and the aromatic redox compound. The liquid ingredients preferably comprise the peroxidic species or reaction products resulting from oxidation of the unsaturated hydrocarbon by the oxygen-containing active agent, along with the penetrating solvent. Administration is preferably intravenously. The reconstituted product preferably may be administered intravenously as a concentrate diluted in saline. Topical, ocular, intraperitoneal, rectal and intrathecal deliveries are also possible routes for administration. Intramuscular injection is not preferred, as it has a tendency to produce local irritation.

Administration of the pharmaceutical formulation *in vivo* is effective in reducing macular drusen buildup and improving the visual acuity of individuals affected with ARMD.

### EXAMPLE 1. OZONOLYSIS OF AN UNSATURATED HYDROCARBON

Ozonolysis of an alkene may be carried out either in a solvent or neat. In either case, the cooling of the reaction mixture is critical in avoiding explosive decomposition of the peroxidic products of the reaction.

The following general procedure is typical for the ozonolysis of a liquid alkene.

A 1-liter flask fitted with a magnetic stirrer is charged with the alkene (2 moles), and the apparatus is weighed. The flask is surrounded by a cooling bath (ice-water or ice-salt). Once the contents are cooled below 5° C, stirring is begun and a stream of ozone in dry oxygen (typically 3% ozone) is passed through the mixture. It is advantageous to disperse the ozonated oxygen through a glass frit, but this is not necessary for a stirred solution. Periodically, the gas stream is stopped, and the reaction flask is weighed or the reaction mixture is sampled. The gas stream is then re-started.

Once the mass of the reaction flask shows sufficient weight gain, or once the proton magnetic resonance ("H¹ NMR") spectrum of the reaction mixture shows the desired reduction in the intensity of the olefinic proton resonances (usually about 50%), the gas flow is stopped.

The ozonolysis may be carried out as above, substituting a solution of the alkene in a solvent non-reactive towards ozone such as saturated hydrocarbons or chlorinated hydrocarbons. The ozonolysis may also be carried out as above, with or without solvent, substituting an alkenol for the alkene without affecting the reaction in any substantive manner.

The reaction mixture is then poured slowly into the cooled penetrating solvent.

### EXAMPLE 2. PREPARATION OF THE PHARMACEUTICAL FORMULATION

A preferred pharmaceutical formulation of the present invention was prepared as follows:
(1) Sparging an ozone/pure oxygen gas mixture of 120 mg/L up through an alkadiene alcohol, 3,7-dimethyl-2,6-octadien-1-ol (geraniol), at 1 Liter of gas per hour;
(2) Maintaining the temperature of the reaction around 5°C;
(3) Removing small aliquots of reaction product hourly and measuring by H¹ NMR the formation of the peroxidic species or reaction products;
(4) Stopping the reaction when more than about 50% of the available unsaturated bonds have been reacted;
(5) Diluting the product mixture with dimethylsulfoxide (1:10) to give a solution or dispersion;
(6) Prior to use in the target biological system, a mixture of hematoporphyrin, rose bengal, and methyl-naphthoquinone dry powders was added to the solution or dispersion in sufficient quantity to create a concentration of 20 micromolar of each component dispersed therein when delivered to the target biological system by saline intravenous infusion. Optionally, ascorbate could be added to the formulation prior to use.

### EXAMPLE 3. EXAMPLES OF THE PHARMACEUTICAL FORMULATION

Two preferred formulations are as follows:
**A.**

| **WEIGHT %** | **INGREDIENT** |
|---|---|
| 0.54* | Tetraoxane dimer of acetal peroxide from ozonation of geraniol |
| 98.00 | DMSO |
| 0.83 | Hematoporphyrin |
| 0.24 | Methylnaphthoquinone |
| 0.39 | Rose Bengal |

| | |
|---|---|
| *Determined by mass spectroscopy. | |

**B.**

| **WEIGHT %** | **INGREDIENT** |
|---|---|
| 0.54* | Tetraoxane dimer of acetal peroxide from ozonation of geraniol |
| 98.00 | DMSO |
| 0.83 | Hematoporphyrin |
| 0.24 | Methylnaphthoquinone |
| 0.39 | Chlorophyllin Sodium-Copper Salt |

| | |
|---|---|
| *Determined by mass spectroscopy. | |

### EXAMPLE 4. EXPERIMENTAL PROCEDURES

To test the effectiveness of the pharmaceutical formulation in reducing macular drusen buildup and restoring visual acuity, 23 patients with documented dry ARMD and no previous history of neovascularization or laser treatment were administered an example of the pharmaceutical formulation. None of the patients were taking zinc supplementation, plasmapheresis or vitamin therapy during the trial or 3 months prior. All patients had a minimum ten-year history of smoking at least one pack per day. All were told to stop smoking for the trial and a one-year follow-up period. Each of the patients had documented atherosclerotic cardiovascular disease. As a pre-clinical consensual pilot trial, ethical concerns prevented the use of a placebo controlled "blinded" population.

Intravenous infusion of the pharmaceutical formulation having the Formulation B from Example 3 above (diluted 1 cc into 100 cc of normal saline) was accomplished over 15 minutes, daily, in sets of three monthly infusions, for 9-12 planned treatments. Follow-up testing of individual eye, best corrected visual acuity was performed at 3 months, 6 months and 1 year.

### EXAMPLE 5. DRUSEN REDUCTION

Photographic and angiographic documentation during and after the treatment period did not express any regeneration of cell components, closure of voids, or late development of neovascularization. In other words, dead cells stayed dead and pigment clumping was unchanged. Drusen, however, disappeared within weeks, as evidenced by fluorescence changes from choroidal autofluorescence by drusen to a hyperfluorescence "window defect" created by the void. Photographic evidence also showed the lessening or disappearance of drusen from the maculas. The visual acuity change did not correlate with any photographic or angiographic appearance. Despite the major acuity improvement, morphological changes of previous cell death and pigment clumping were static and permanent. However, the results do indicate that the pharmaceutical formulation is effective at reducing drusen.

### EXAMPLE 6. VISUAL ACUITY IMPROVEMENT

Table 6-1 below summarizes the mean return of visual acuity performance following treatment, as noted in decimal (non-Snellen) form. As shown in the table, the patients showed a mean 48.05% improvement in visual acuity 1 year after treatment.

**Table 6 -1. Mean Visual Acuity**

| | Pre-Treatment | 3 Months | 6 Months | 12 Months |
|---|---|---|---|---|
| First eye | 0.27 | 0.63 | 0.66 | 0.72 |
| Second eye | 0.22 | 0.63 | 0.65 | 0.73 |
| Both eyes | 0.24 | 0.63 | 0.65 | 0.72 |
| Overall percentage gain | --- | 38.95% | 41.30% | 48.05% |

### REFERENCES CITED

### U.S. Patents

U.S. Patent No. 4,451,480 to DeVillez
U.S. Patent No. 4,591,602 to DeVillez
U.S. Patent No. 4,983,637 to Herman
U.S. Patent No. 5,086,076 to Herman
U.S. Patent No. 5,126,376 to Herman
U.S. Patent No. 5,190,977 to Herman
U.S. Patent No. 5,190,979 to Herman
U.S. Patent No. 5,260,342 to Herman
U.S. Patent No. 5,270,344 to Herman
U.S. Patent No. 5,364,879 to Herman

### Other Publications

DalleDonne, I., Milzani, A., Colombo, R. Actin assembly by cadmium ions. Biochim Biophys Acta vol. 1357(1), pp. 5 - 17, 1997.
Levy, L., Vredevoe, D.L., Cook, G. In vitro reversibility of cadmium-induced inhibition of phagocytosis. Environ Res vol. 41(2), pp. 361 - 71, 1986.

## Claims

1. Use of:
a peroxidic species or reaction products resulting from oxidation of menthol or an alkene by an oxygen-containing oxidizing agent, wherein the alkene comprises terpineol, citronellol, nerol, linalool, phytol, geraniol, perillyl alcohol, menthol, geranylgeraniol or farnesol;
a penetrating solvent, wherein the penetrating solvent comprises dimethylsulfoxide, sterol, lecithin, propylene glycol, or methylsulfonylmethane;
a dye containing a chelated divalent or trivalent metal, wherein the dye comprises porphyrin, rose bengal, chlorophyllin, hemin, corrins, texaphrin, methylene blue, hematoxylin, eosin, erythrosin, lactoflavin, anthracene dye, hypericin, methylcholanthrene, neutral red, phthalocyanine, fluorescein, eumelanin, or pheomelanin; and
an aromatic redox compound, wherein the redox compound comprises substituted or unsubstituted benzoquinone, naphthoquinone, or anthroquinone,
in the manufacture of a pharmaceutical formulation for treating age-related macular degeneration ("ARMD"),
wherein the peroxidic species or reaction products resulting from oxidation of menthol or the alkene is from 0.001% to 30% by weight of the pharmaceutical formulation,
wherein the penetrating solvent is from 50% to 99% by weight of the pharmaceutical formulation,
wherein the dye is from 0.1% to 30% by weight of the pharmaceutical formulation, and
wherein the aromatic redox compound is from 0.01% to 20% by weight of the pharmaceutical formulation.

2. The use of claim 1, wherein the alkene is in a liquid form, in a solution, or in a dispersion.

3. The use of claim 1, wherein the alkene is contained in a fixed oil, an ester, a fatty acid, or an ether.

4. The use of claim 1, wherein the oxygen-containing oxidizing agent comprises singlet oxygen, oxygen in its triplet state, superoxide anion, periodate, hydroxyl radical, hydrogen peroxide, alkyl peroxide, carbamyl peroxide, benzoyl peroxide, or oxygen bound to a transition element.

5. The use of claim 1, wherein the oxygen-containing oxidizing agent comprises ozone.

6. The use of claim 1, wherein the penetrating solvent is a liquid, micelle membrane, liposome, emollient, or vapor.

7. The use of claim 1, wherein the penetrating solvent is dimethylsulfoxide ("DMSO").

8. The use of claim 1, wherein the dye comprises porphyrin, rose bengal, or a mixture thereof.

9. The use of claim 1, wherein the metal comprises iron.

10. The use of claim 1, wherein the metal comprises copper, manganese, tin, magnesium, or strontium.

11. The use of claim 1, further comprising an electron donor.

12. The use of claim 11, wherein the electron donor comprises ascorbic acid or a pharmaceutical salt thereof.

13. Use according to claim 1, wherein the pharmaceutical formulation comprises:
a peroxidic species or reaction products resulting from oxidation of geraniol by a mixture of ozone and oxygen;
dimethylsulfoxide ("DMSO");
a dye containing a chelated divalent or trivalent metal, wherein the dye comprises a mixture of hematoporphyrin and rose bengal or a mixture of hematoporphyrin and chlorophyllin; and
methylnaphthoquinone.

## Patentansprüche

1. Verwendung von:
einer peroxidischen Spezies oder Reaktionsprodukten, die aus der Oxidation von Menthol oder einem Alken durch ein sauerstoffhaltiges oxidierendes Mittel stammen, wobei das Alken Terpineol, Citronellol, Nerol, Linalool, Phytol, Geraniol, Perillylalkohol, Menthol, Geranylgeraniol oder Farnesol umfasst;
ein Penetrierlösungsmittel, wobei das Penetrierlösungsmittel Dimethylsulfoxid, Sterin, Lecithin, Propylenglykol oder Methylsulfonylmethan umfasst;
ein Farbstoff, der ein cheliertes zweiwertiges oder dreiwertiges Metall enthält, wobei der Farbstoff Porphyrin, Bengalrosa, Chlorophyllin, Hämin, Corrine, Texaphrin, Methylenblau, Hämatoxylin, Eosin, Erythrosin, Lactoflavin, Anthracenfarbstoff, Hypericin, Methylcholanthren, Neutralrot, Phthalocyanin, Fluorescein, Eumelanin oder Pheomelanin umfasst; und
eine aromatische Redoxverbindung, wobei die Redoxverbindung substituiertes oder nicht substituiertes Benzochinon, Naphthochinon oder Anthrochinon umfasst,
zur Herstellung einer pharmazeutischen Formulierung für die Behandlung von altersbedingter Makuladegeneration ("AMD"),
wobei die peroxidische Spezies oder Reaktionsprodukte, die aus der Oxidation von Menthol oder einem Alken stammen, von 0,001 Gew.-% bis 30 Gew.-% der pharmazeutischen Formulierung betragen,
wobei das Penetrierlösungsmittel 50 Gew.-% bis 99 Gew.-% der pharmazeutischen Formulierung beträgt,
wobei der Farbstoff 0,1 Gew.-% bis 30 Gew.-% der pharmazeutischen Formulierung beträgt, und
wobei die aromatische Redoxverbindung 0,01 Gew.-% bis 20 Gew.-% der pharmazeutischen Formulierung beträgt.

2. Die Verwendung nach Anspruch 1, wobei das Alken in einer flüssigen Form, als eine Lösung oder als eine Dispersion vorliegt.

3. Die Verwendung nach Anspruch 1, wobei das Alken in einem festen Öl, in einem Ester, in einer Fettsäure oder in einem Ether enthalten ist.

4. Die Verwendung nach Anspruch 1, wobei das sauerstoffhaltige oxidierende Mittel Singulettsauerstoff, Sauerstoff in seinem Triplettzustand, Superoxidanion, Periodat, Hydroxylradikal, Wasserstoffperoxid, Alkylperoxid, Carbamylperoxid, Benzoylperoxid, oder an ein Übergangselement gebundenen Sauerstoff umfasst.

5. Die Verwendung nach Anspruch 1, wobei das sauerstoffhaltige oxidierende Mittel Ozon umfasst.

6. Die Verwendung nach Anspruch 1, wobei das Penetrierlösungsmittel eine Flüssigkeit, Mizellenmembran, Liposom, Weichmacher oder Dampf ist.

7. Die Verwendung nach Anspruch 1, wobei das Penetrierlösungsmittel Dimethylsulfoxid ("DMSO") ist.

8. Die Verwendung nach Anspruch 1, wobei der Farbstoff Porphyrin, Bengalrosa oder ein Gemisch davon umfasst.

9. Die Verwendung nach Anspruch 1, wobei das Metall Eisen umfasst.

10. Die Verwendung nach Anspruch 1, wobei das Metall Kupfer, Mangan, Zinn, Magnesium oder Strontium umfasst.

11. Die Verwendung nach Anspruch 1, weiterhin umfassend einen Elektronendonor.

12. Die Verwendung nach Anspruch 11, wobei der Elektronendonor Ascorbinsäure oder ein pharmazeutisches Salz davon umfasst.

13. Die Verwendung gemäß Anspruch 1, wobei die pharmazeutische Formulierung umfasst:
eine peroxidische Spezies oder Reaktionsprodukte, die aus der Oxidation von Geraniol durch eine Mischung aus Ozon und Sauerstoff stammen;
Dimethylsulfoxid ("DMSO");
ein Farbstoff, der ein cheliertes zweiwertiges oder dreiwertiges Metall enthält, wobei der Farbstoff eine Mischung aus Hämatoporphyrin und Bengalrosa oder eine Mischung aus Hämatoporphyrin und Chlorophyllin umfasst; und
Methylnaphthochinon.

## Revendications

1. Utilisation :
d'une espèce de type peroxyde ou de produits de réaction résultant de l'oxydation du menthol ou d'un alcène par un agent oxydant contenant de l'oxygène, l'alcène comprenant le terpinéol, le citronellol, le nérol, le linalool, le phytol, le géraniol, l'alcool périllylique, le menthol, le géranylgéraniol ou le famésol ;
d'un solvant pénétrant, le solvant pénétrant comprenant le diméthylsulfoxyde, le stérol, la lécithine, le propylèneglycol ou le méthylsulfonylméthane ;
d'un colorant contenant un métal divalent ou trivalent chélaté, le colorant comprenant la porphyrine, le rose Bengale, la chlorophylline, l'hémine, les corrines, la texaphrine, le bleu de méthylène, l'hématoxyline, l'éosine, l'érythrosine, la lactoflavine, un colorant anthracénique, l'hypéricine, le méthylcholanthrène, le rouge neutre, la phtalocyanine, la fluorescéine, l'eumélanine ou la phéomélanine ; et
d'un composé redox aromatique, le composé redox comprenant la benzoquinone, la naphtoquinone ou l'anthraquinone, substituées ou non substituées,
dans la fabrication d'une composition pharmaceutique destinée au traitement de la dégénérescence maculaire liée à l'âge (DMLA),
dans laquelle l'espèce de type peroxyde ou les produits de réaction résultant de l'oxydation du menthol ou d'un alcène est(sont) contenu(s) à raison de 0,001 % à 30 % en poids de la composition pharmaceutique,
dans laquelle le solvant pénétrant est contenu à raison de 50 % à 99 % en poids de la composition pharmaceutique,
dans laquelle le colorant est contenu à raison de 0,1 % à 30 % en poids de la composition pharmaceutique et
dans laquelle le composé redox aromatique est contenu à raison de 0,01 % à 20 % en poids de la composition pharmaceutique.

2. Utilisation selon la revendication 1, dans laquelle l'alcène est sous une forme liquide, en une solution ou en une dispersion.

3. Utilisation selon la revendication 1, dans laquelle l'alcène est contenu dans une huile fixe, un ester, un acide gras ou un éther.

4. Utilisation selon la revendication 1, dans laquelle l'agent oxydant contenant de l'oxygène comprend l'oxygène singulet, l'oxygène en son état triplet, un anion superoxyde, un periodate, le radical hydroxy, le peroxyde d'hydrogène, un peroxyde d'alkyle, le peroxyde de carbamyle, le peroxyde de benzoyle ou une liaison oxygène à un élément de transition.

5. Utilisation selon la revendication 1, dans laquelle l'agent oxydant contenant de l'oxygène comprend l'ozone.

6. Utilisation selon la revendication 1, dans laquelle le solvant pénétrant est un liquide, une membrane micellaire, un liposome, un émollient ou une vapeur.

7. Utilisation selon la revendication 1, dans laquelle le solvant pénétrant est le diméthylsulfoxyde (DMSO).

8. Utilisation selon la revendication 1, dans laquelle le colorant comprend la porphyrine, le rose Bengale ou un mélange de ceux-ci.

9. Utilisation selon la revendication 1, dans laquelle le métal comprend le fer.

10. Utilisation selon la revendication 1, dans laquelle le métal comprend le cuivre, le manganèse, l'étain, le magnésium ou le strontium.

11. Utilisation selon la revendication 1, comprenant en outre un donneur d'électrons.

12. Utilisation selon la revendication 11, dans laquelle le donneur d'électron comprend un acide ascorbique ou un sel pharmaceutique de celui-ci.

13. Utilisation selon la revendication 1, dans laquelle la composition pharmaceutique comprend
une espèce de type peroxyde ou des produits de réaction résultant de l'oxydation du géraniol par un mélange d'ozone et d'oxygène ;
du diméthylsulfoxyde (DMSO) ;
un colorant contenant un métal divalent ou trivalent chélaté, le colorant comprenant un mélange d'hématoporphyrine et de rose Bengale ou un mélange d'hématoporphyrine et de chlorophylle ; et
de la méthylnaphtoquinone.
